# FASCICULE DE BREVET EUROPEEN

(11) **EP 0 852 153 B1**
(45) Date de publication et mention de la délivrance du brevet: **03.09.2003**
(21) Numéro de dépôt: 98400002.6
(22) Date de dépôt: 05.01.1998
(51) Int. Cl.: A61M 16/00, A61M 16/04

(54) **Dispositif pour échanger des sondes respiratoires dans la trachée d'un patient**
Vorrichtung zum Wechseln eines Beatmungstubus in der Trachea eines Patienten
Device for changing respiratory tubes in a patient's trachea

(30) Priorité: 06.01.1997 FR 9700043
(43) Date de publication de la demande: 08.07.1998
(73) Titulaire: Boussignac, Georges, 92160 Antony (FR)
(72) Inventeur: Boussignac, Georges, 92160 Antony (FR)
(74) Mandataire: Bonnetat, Christian

(56) Documents cités:
- WO-A-90/04992
- US-A- 4 244 362
- US-A- 4 960 122
- US-A- 5 052 386

## Description

La présente invention concerne un dispositif permettant de remplacer une première sonde respiratoire, par exemple usagée, déjà en place dans la trachée d'un patient par une seconde sonde respiratoire, par exemple neuve, extérieure audit patient. Un tel dispositif est divulgué dans le document US-A-5 052 386.

On sait que les patients nécessitant une assistance respiratoire sont reliés à une source de gaz appropriée, par exemple associée à un respirateur artificiel.

Le patient est pourvu, dans sa trachée, d'une sonde et cette sonde est reliée à ladite source de gaz respiratoire par au moins une conduite d'alimentation.

On sait de plus que, pour des raisons d'hygiène ou pour des raisons techniques, une telle sonde doit être remplacée de temps à autre, par exemple à cause de la dégradation de certains de ses éléments (ballonnet de maintien en place), ou bien pour remplacer la sonde en place par une sonde de type différent. Généralement, la mise en place d'une sonde de remplacement est une opération complexe et douloureuse, du fait que bien souvent la surface de la trachée est tuméfiée et que le diamètre de celle-ci est réduit.

De plus, le processus de remplacement d'une sonde par une autre nécessite l'interruption de l'assistance respiratoire pendant un temps relativement long, ce qui est préjudiciable à la santé du patient.

La présente invention a pour objet de remédier à ces inconvénients par le dispositif, comme defini dans la revendication 1, pour remplacer une première sonde respiratoire déjà en place dans la trachée d'un patient par une seconde sonde respiratoire extérieure audit patient. Par ailleurs, il est avantageux que lesdites sondes respiratoires sont aptes à glisser sur ladite conduite d'alimentation auxiliaire.

Ainsi, grâce à la présente invention, le processus pour remplacer une première sonde respiratoire déjà en place dans la trachée d'un patient par une seconde sonde respiratoire extérieure audit patient, ladite première sonde étant alimentée en gaz d'assistance respiratoire par au moins une conduite d'alimentation principale connectée à ladite première sonde, peut consister en la suite des étapes suivantes :
a) on déconnecte ladite conduite d'alimentation principale de ladite première sonde respiratoire et on introduit, dans ladite première sonde respiratoire, ledit dispositif tubulaire souple et allongé, présentant au moins un orifice distal et au moins un orifice proximal ;
b) on connecte, par son orifice proximal, ledit dispositif tubulaire souple et allongé à ladite conduite auxiliaire d'alimentation en gaz respiratoire à pression plus élevée que celui de la conduite d'alimentation principale ;
c) on extrait ladite première sonde de ladite trachée, en la faisant glisser le long dudit dispositif tubulaire souple et allongé maintenu en place dans la trachée, et, éventuellement, le long de ladite conduite d'alimentation auxiliaire ;
d) on déconnecte ladite conduite d'alimentation auxiliaire dudit dispositif tubulaire souple et allongé et on élimine ladite première sonde ;
e) on enfile ladite seconde sonde sur ladite conduite d'alimentation auxiliaire, ou éventuellement, sur ledit dispositif tubulaire souple et allongé et on connecte, par son orifice proximal, ledit dispositif tubulaire souple et allongé à ladite conduite d'alimentation auxiliaire ; et
f) on fait glisser ladite seconde sonde sur ledit dispositif tubulaire souple et allongé jusqu'à mise en place correcte dans la trachée.

On peut donc, grâce à la présente invention, minimiser l'interruption d'assistance respiratoire puisque chaque déconnexion des conduites d'alimentation peut être de courte durée. De plus, ledit dispositif tubulaire souple et allongé sert de guide pour l'extraction de la première sonde respiratoire et pour la mise en place de la seconde desdites sondes, ce qui facilite l'échange des sondes et en réduit le processus opératoire.

On remarquera que, par ailleurs, ledit dispositif tubulaire souple et allongé peut servir de guide lors de la mise en place antérieure de ladite première sonde. Il pourrait également, après l'étape f), rester en place dans ladite seconde sonde pour dispenser le gaz d'assistance respiratoire au patient.

Cependant, selon un mode de mise en oeuvre avantageux du processus décrit ci-dessus, après l'étape f) susmentionnée, on procède aux deux étapes successives additionnelles suivantes :
g) on déconnecte ladite conduite d'alimentation auxiliaire dudit dispositif tubulaire souple et allongé et on extrait ledit dispositif tubulaire souple et allongé de ladite seconde sonde ; et
h) on connecte ladite conduite d'alimentation principale à ladite seconde sonde.

Le dispositif conforme à la présente invention peut comporter :
- une âme tubulaire centrale ayant un orifice proximal apte à être relié à ladite conduite d'alimentation auxiliaire et un orifice distal pour dispenser ledit gaz respiratoire ; et
- une gaine tubulaire, entourant ladite âme tubulaire avec jeu et solidaire de ladite âme tubulaire centrale.

De préférence, ladite gaine tubulaire est, à son extrémité distale, en saillie par rapport à l'extrémité distale de ladite âme tubulaire.

Ainsi, ladite âme tubulaire centrale est protégée par ladite gaine tubulaire de sorte que l'orifice distal de ladite âme tubulaire ne peut pas être obturé, par exemple par des mucosités. Pour des raisons semblables, dans un premier mode de réalisation, ladite gaine tubulaire est pourvue d'orifices dans sa paroi latérale et est fermée, à son extrémité distale, par une paroi d'obturation, éventuellement pourvue d'un ou de plusieurs orifices.

Avantageusement, la longueur dudit dispositif est alors telle que, lorsqu'il est mis en place dans la trachée dudit patient, ladite gaine tubulaire sort à l'extérieur de la bouche de celui-ci et la partie de ladite gaine, extérieure audit patient, est également pourvue d'orifices.

Ainsi, il ne peut se produire de surpression dangereuse dans la trachée du patient puisque le gaz d'assistance respiratoire peut s'évacuer par les orifices de la gaine extérieure au patient.

Dans un deuxième mode de réalisation dudit dispositif conforme à la présente invention, la gaine tubulaire est ouverte à son extrémité distale et ledit jeu, compris entre l'âme centrale et la gaine, est relié à une source de fluide sous pression, par exemple une source d'eau. Il est ainsi possible d'humidifier la trachée et d'éviter le dessèchement de celle-ci sous l'action du gaz d'assistance respiratoire. Bien entendu, tout autre fluide (médicament par exemple) pourrait être ainsi véhiculé à travers ledit jeu. De plus, il est possible de prévoir des conduits auxiliaires de fluide entre ladite gaine et ladite âme, ainsi qu'éventuellement dans l'épaisseur de paroi de celles-ci.

De plus, pour éviter l'écrasement de l'âme et de la gaine à l'emplacement des coudes du dispositif (et donc l'interruption de l'assistance respiratoire), ainsi que pour centrer ledit dispositif par rapport aux parois de la trachée, il est avantageux que ledit dispositif, quel que soit son mode de réalisation, comporte un ressort hélicoidal extérieur, entourant ladite gaine tubulaire. Un tel ressort peut être en une matière à mémoire de forme (Nitinol, par exemple) et ne prendre sa forme hélicoïdale que lorsqu'il est soumis aux conditions de température régnant dans la trachée.

Selon un troisième mode de réalisation dudit dispositif conforme à la présente invention, ladite âme tubulaire centrale et ladite gaine tubulaire présentent une forme hélicoïdale.

Dans encore un autre mode de réalisation, le dispositif conforme à l'invention comporte un corps allongé, par exemple en matière synthétique, pourvu d'une pluralité de canaux longitudinaux, dont l'un est apte à être relié à une conduite d'alimentation auxiliaire en gaz respiratoire, à son extrémité proximale. Dans un autre desdits canaux longitudinaux peut être introduit un fil déformable, afin de donner sa forme, par exemple hélicoïdale, audit dispositif.

Les figures du dessin annexé feront bien comprendre comment l'invention peut être réalisée. Sur ces figures, des références identiques désignent des éléments semblables.
La figure 1 est une vue schématique illustrant un patient à respiration assistée.
Les figures 2a à 2i illustrent le processus d'échange de sondes d'assistance respiratoire, au moyen du dispositif conforme à la présente invention.
La figure 3 illustre en coupe longitudinale axiale un premier mode de réalisation du dispositif conforme à la présente invention.
La figure 4 est une coupe transversale selon la ligne IV-IV de la figure 3.
La figure 5 illustre une variante de réalisation du dispositif conforme au premier mode de la présente invention, également en coupe transversale semblable à la figure 4.
Les figures 6 et 7 illustrent, en vues longitudinales schématiques, deux autres modes de réalisation du dispositif conforme à la présente invention.
Les figures 8 et 9 montrent deux variantes du mode de réalisation de la figure 7.
La figure 10 illustre, en coupe transversale, encore un autre mode de réalisation du dispositif conforme à la présente invention.

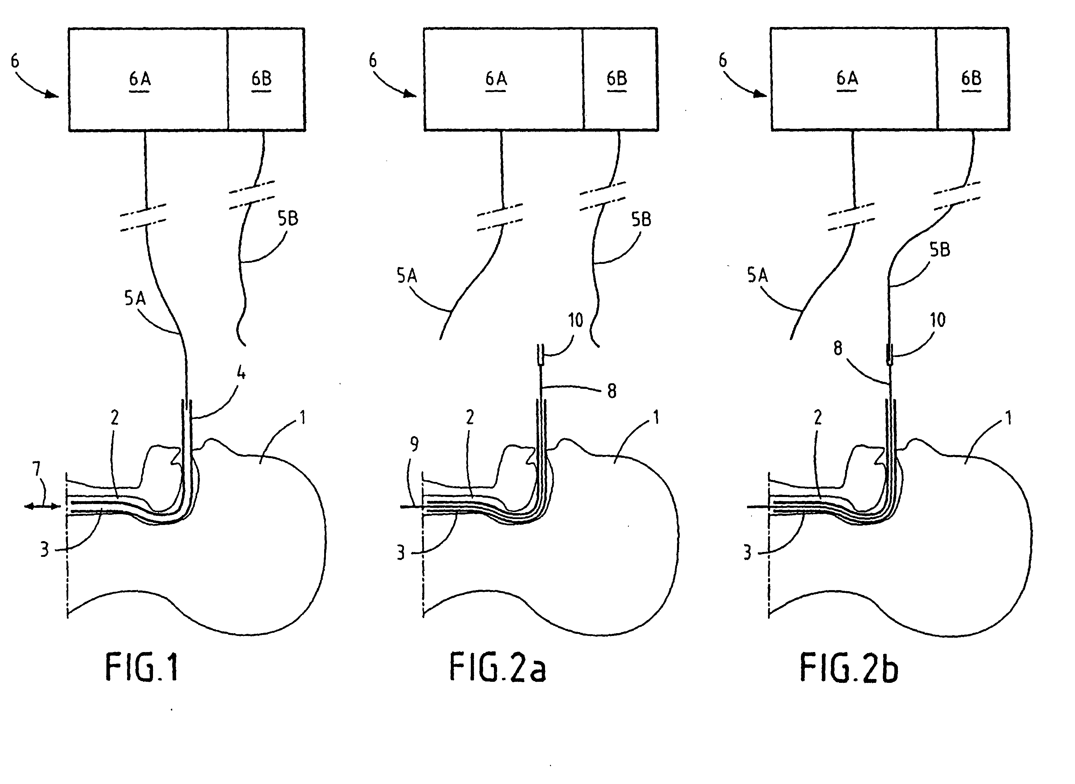

Sur la figure 1, on a représenté un patient 1 dans la trachée 2 duquel est placée une première sonde respiratoire tubulaire 3, dont l'extrémité proximale 4 sort à l'extérieur de la bouche dudit patient. Sur la figure 1, la sonde est représentée de façon extrêmement schématique, sous la forme d'un simple tube, et on n'y a pas représenté les moyens de maintien de la sonde 3 (tels qu'un ballonnet) dans la trachée 2.

L'extrémité proximale 4 de la sonde 3 est reliée par une conduite d'alimentation 5A à un respirateur artificiel 6A. Ainsi, le respirateur artificiel 6A peut adresser aux poumons du patient 1 des cycles de gaz respiratoire (voir les flèches 7), à une pression appropriée.

Comme on peut le voir sur les figures 1 à 2i, le respirateur artificiel 6A est associé à une source de gaz respiratoire auxiliaire 6B pour former une double source gazeuse 6. La source auxiliaire 6B délivre, par l'intermédiaire d'une conduite d'alimentation auxiliaire 5B, du gaz respiratoire à une pression supérieure à celui délivré par la conduite principale 5A.

Lorsque la sonde doit être remplacée, on commence par déconnecter la conduite d'alimentation principale 5A de ladite première sonde respiratoire 3 et on introduit dans celle-ci un dispositif tubulaire souple et allongé 8, conforme à la présente invention et présentant au moins un orifice à son extrémité distale 9 et au moins un orifice à son extrémité proximale 10 (figure 2a). Ensuite, la conduite auxiliaire 5B est reliée à l'orifice de l'extrémité proximale dudit dispositif tubulaire 8, de sorte que l'interruption d'assistance respiratoire du patient est courte et juste suffisante pour introduire l'élément tubulaire souple dans la sonde 3 (voir la figure 2b). Grâce au fait que la pression de la source 6B est supérieure à celle du respirateur 6A, l'assistance respiratoire est satisfaisante malgré le plus faible diamètre du dispositif tubulaire 8.

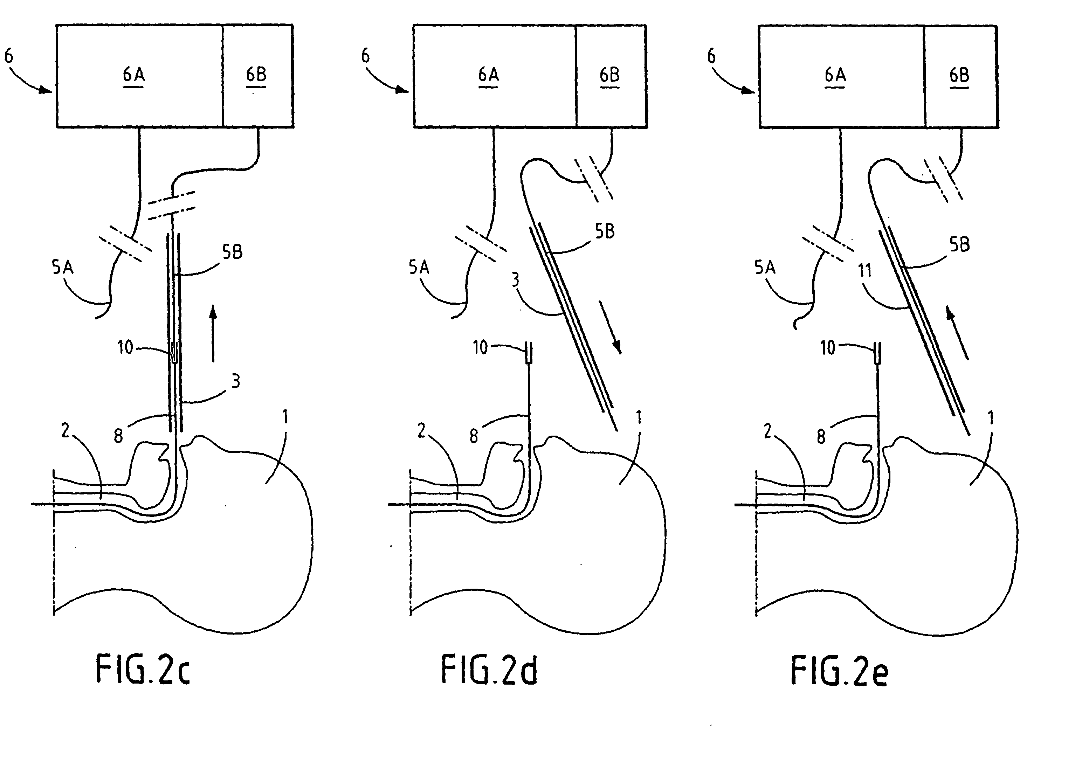

Ensuite (voir la figure 2c), la première sonde 3 est extraite de la trachée 2 du patient en la faisant glisser le long dudit dispositif tubulaire 8 et de ladite conduite d'alimentation auxiliaire 5B.

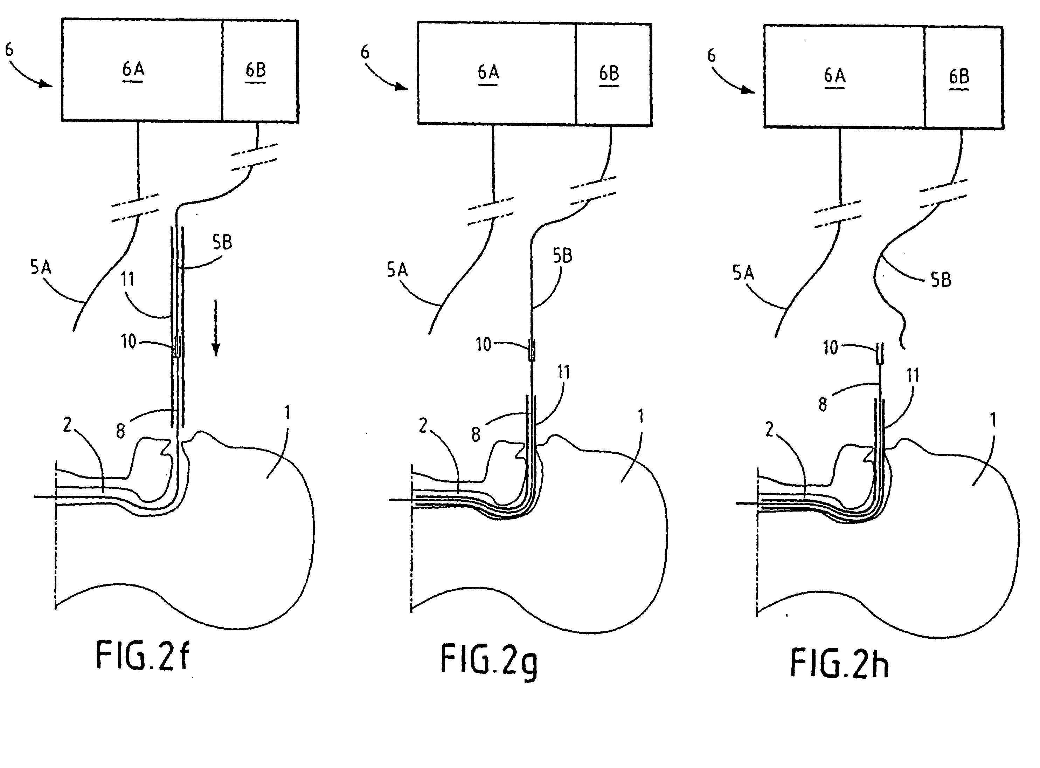

Il est alors possible (voir la figure 2d) de débrancher la conduite auxiliaire 5B de l'extrémité proximale 10 du dispositif tubulaire souple 8 et d'éliminer ladite sonde 3. Celle-ci peut être remplacée par une seconde sonde 11 (figure 2e) enfilée sur ladite conduite auxiliaire 5B. On reconnecte par la suite l'extrémité proximale 10 du dispositif tubulaire souple 8 à la conduite auxiliaire 5B et on fait glisser la nouvelle sonde respiratoire 11 dans la trachée 2 du patient 1, en se servant dudit dispositif tubulaire souple 8 comme d'un guide de mise en place (figure 2f).

Là encore, on constate que l'interruption de l'assistance respiratoire est très courte, juste suffisante pour remplacer la première sonde 3 par la seconde sonde 11, sur la conduite d'alimentation auxiliaire 5B.

Enfin (voir la figure 2g), la seconde sonde respiratoire 11 est poussée sur le guide 8 jusqu'à prendre la position de la première sonde 2.

Eventuellement, on déconnecte ensuite le dispositif tubulaire souple 8 de la conduite auxiliaire 5B (figure 2h), on extrait ledit dispositif tubulaire souple 8 et on connecte la conduite d'alimentation principale 5A à l'extrémité proximale 12 de ladite seconde sonde 11 (figure 2i).

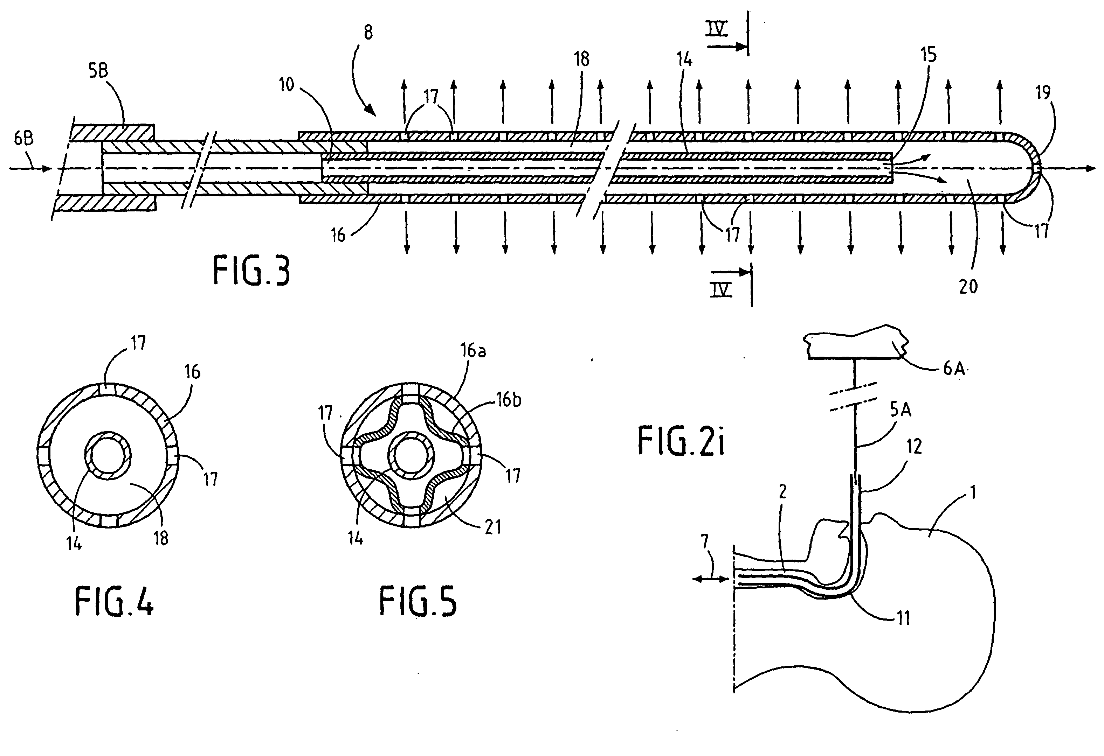

Sur les figures 3 et 4, on a représenté un premier mode de réalisation pour le dispositif tubulaire souple et allongé 8. Comme on peut le voir, celui-ci comporte une âme tubulaire centrale 14 comportant un orifice proximal 10 destiné à être relié à la conduite auxiliaire 5B d'alimentation en gaz respirable et un orifice distal 15 destiné à dispenser le gaz d'assistance respiratoire amené par la conduite d'alimentation auxiliaire 5B.

L'âme centrale 14 est entourée par une âme tubulaire 16 pourvue d'orifices 17 dans sa paroi latérale. Entre l'âme centrale 14 et la gaine tubulaire 16, est ménagé un espace 18 de section annulaire.

A son extrémité distale, la gaine tubulaire 16 est obturée par une paroi 19 elle-même pourvue d'orifices 17.

Comme on peut le voir, l'extrémité distale 15 de l'âme tubulaire centrale 14 est en retrait par rapport à la paroi d'extrémité 19 de la gaine tubulaire 16, de sorte qu'entre l'orifice distal 15 et la paroi 19 est ménagée une chambre 20.

Par ailleurs, la longueur du dispositif 8 est telle que les orifices 17, qui se trouvent du côté proximal de la gaine 16, se trouvent à l'extérieur de la bouche du patient lorsque l'élément tubulaire 8 est en place dans les sondes 3 et 11.

Ainsi, le gaz amené par la conduite 5 est véhiculé par l'âme tubulaire 14 jusque dans la chambre 20 puis, de celle-ci, dans l'espace annulaire 18 et à travers les orifices 17 vers les poumons du patient.

Bien entendu, bien que sur les figures 3 et 4 on ait représenté un mode de réalisation particulièrement simple, il est possible de compléter le dispositif tubulaire 8 en lui adjoignant des canaux internes, pour l'injection de médicament, la prise de pression, etc ...

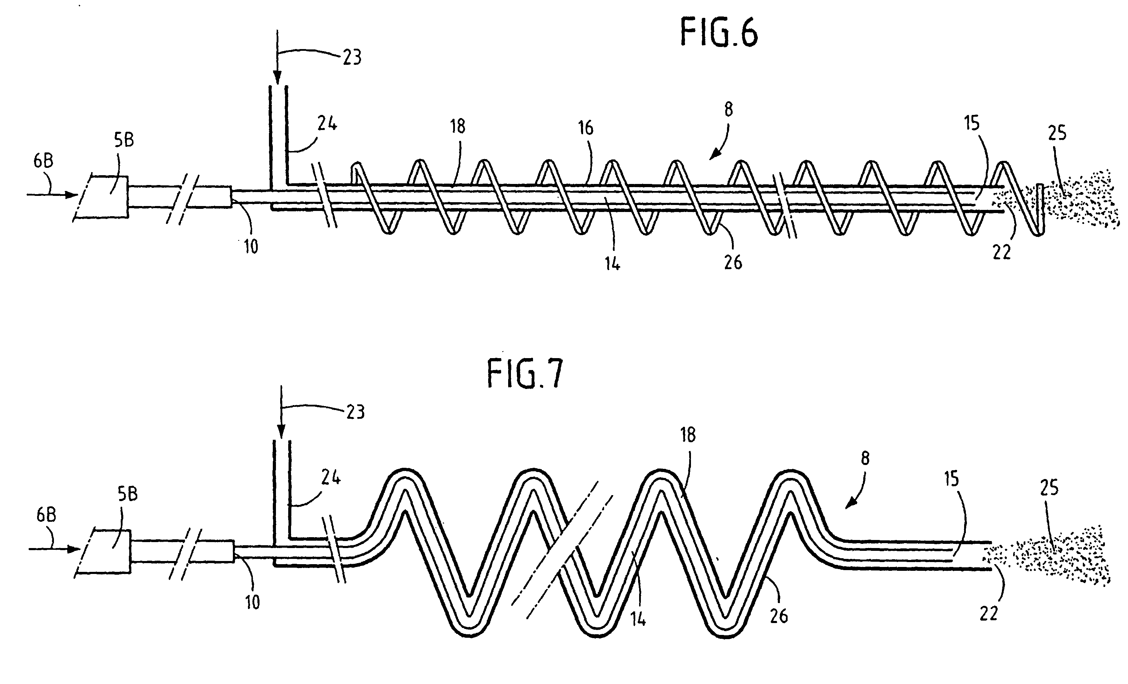

Sur la figure 5, on a représenté une variante de réalisation dans laquelle la gaine 16 comporte deux parois 16a et 16b, solidaires l'une de l'autre uniquement au voisinage des trous 17, pour ménager entre elles un canal 21 destiné soit à introduire un médicament, soit à mesurer une pression, ou encore à tout autre usage.

Le dispositif tubulaire souple 8 est, de préférence, réalisé en une matière synthétique.

Dans le deuxième mode de réalisation du dispositif tubulaire souple et allongé 8, montré par la figure 6, l'enveloppe 16 est ouverte à son extrémité distale par un orifice 22 et sa paroi latérale ne comporte aucun orifice 17.

A son extrémité proximale, l'espace 18 à section annulaire est relié à une source de fluide sous pression 23, par exemple de l'eau, par l'intermédiaire d'un raccord 24.

Ainsi, à la sortie du dispositif 8 apparaît un nuage d'eau nébulisée 25 entraîné par le courant de gaz d'assistance respiratoire traversant l'âme 14.

De plus, afin d'éviter les écrasements de l'âme 14 et de l'enveloppe 16 dans les coudes faits par le dispositif 8, celui-ci comporte un ressort hélicoïdal 26, solidaire d'au moins l'extrémité proximale de l'enveloppe 16 et entourant celle-ci. Le diamètre du fil de l'hélice 26 est par exemple de l'ordre de 300 à 600 microns. Le ressort hélicoïdal 26 est par exemple réalisé en matière à mémoire de forme, telle que celle connue sous le nom commercial de NITINOL.

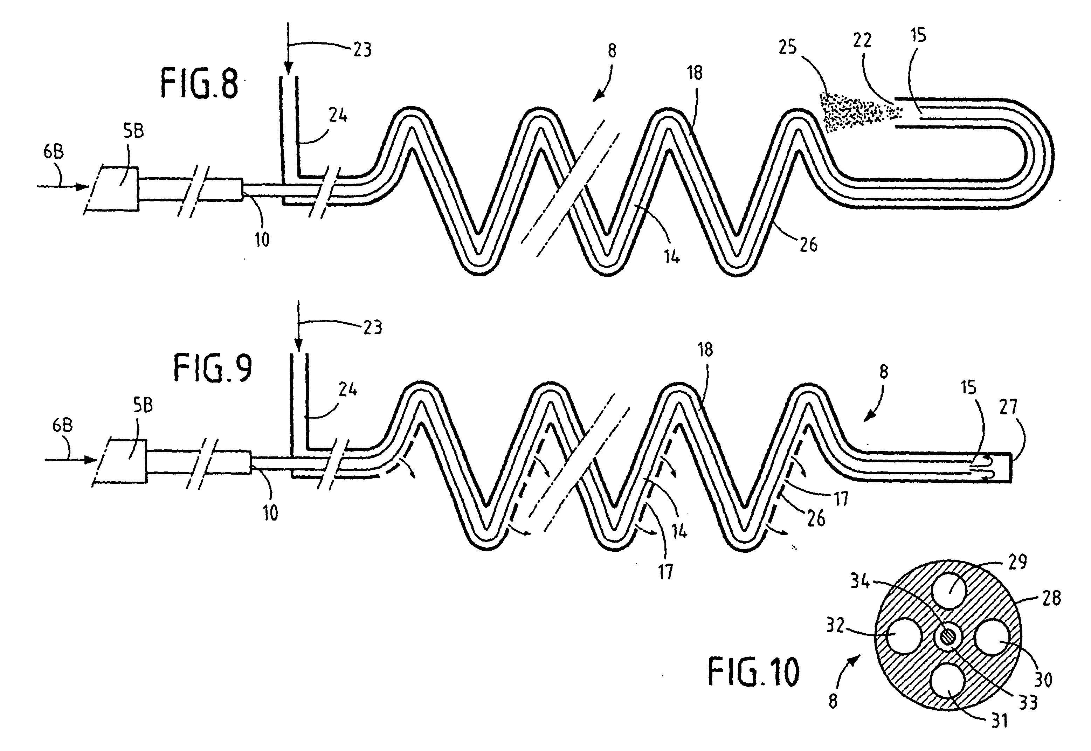

Selon le troisième mode de réalisation illustré par la figure 7, l'âme 14 et l'enveloppe 16 forment chacune une hélice, l'hélice de l'âme 14 étant logée dans l'hélice de l'enveloppe 16. On obtient donc ainsi la protection contre l'écrasement, sans avoir à prévoir un ressort hélicoïdal 26 particulier. On remarquera, comme cela est montré par la figure 8, que l'extrémité distale du dispositif 8 peut présenter toute orientation souhaitée. Sur cette figure 8, ladite extrémité distale est recourbée vers l'extrémité proximale.

Par ailleurs, dans la variante de réalisation de la figure 9, l'orifice 22 de l'extrémité distale de l'enveloppe 16 est fermée par une paroi 27, tandis que la paroi de ladite enveloppe 16 comporte des orifices 17, répartis sur sa longueur et, par exemple, dirigés vers l'axe des hélices 14 et 16. On répartit ainsi l'injection du gaz respirable le long du dispositif 8.

La figure 10 illustre, en coupe transversale, un autre exemple de réalisation pour le corps allongé 28 du dispositif 8. Ce corps 28 comporte une pluralité de canaux longitudinaux 29 à 33, dont deux peuvent jouer le rôle de l'âme 14 et de l'espace annulaire 18 décrits ci-dessus. D'autres canaux longitudinaux peuvent servir à l'injection de médicaments, à la mesure de pression, etc ... Un dernier canal longitudinal 33 est traversé par un fil déformable 34, par exemple métallique, permettant de conformer ledit corps 28 (par exemple en hélice).

## Revendications

1. Dispositif pour remplacer une première sonde respiratoire (3) déjà en place dans la trachée (2) d'un patient (1) par une seconde sonde respiratoire (11) extérieure audit patient, ladite première sonde (3) étant alimentée en gaz d'assistance respiratoire par au moins une conduite d'alimentation principale (5A),
**caractérisé en ce qu'**il comporte:
- une conduite auxiliaire (5B) d'alimentation en gaz respiratoire à une pression plus élevée que celui de ladite conduite d'alimentation principale (5A) ; et
- un élément tubulaire (8), souple et allongé et présentant au moins un orifice distal (9) et au moins un orifice proximal (10):
• qui est apte à être introduit dans lesdites première et seconde sondes respiratoires (3 et 11) en permettant à celles-ci de glisser sur ledit élément tubulaire (8),
• dont l'extrémité proximale (10) est apte à être connectée à ladite conduite auxiliaire (SB), et
• dont l'extrémité distale (9) est apte à dispenser le gaz respiratoire de ladite conduite auxiliaire (5B).

2. Dispositif selon la revendication 1,
**caractérisé en ce que** lesdites sondes respiratoires (3 et 11) sont aptes à glisser sur ladite conduite d'alimentation auxiliaire (5B).

3. Dispositif selon l'une des revendications 1 ou 2,
**caractérisé en ce que** ledit élément tubulaire (8) comporte :
- une âme tubulaire centrale (14) ayant l'orifice proximal (10) apte à être relié à ladite conduite d'alimentation auxiliaire (5B) et un orifice distal (15) pour dispenser ledit gaz respiratoire ; et
- une gaine tubulaire (16), entourant ladite âme tubulaire (14) avec jeu (18) et solidaire de ladite âme tubulaire (14).

4. Dispositif selon la revendication 3,
**caractérisé en ce que**, à son extrémité distale, ladite gaine (16) est en saillie par rapport à l'extrémité distale (15) de ladite âme tubulaire (14).

5. Dispositif selon l'une des revendications 3 ou 4,
**caractérisé en ce que** ladite gaine tubulaire (16) est pourvue d'orifices (17) dans sa paroi latérale et est fermée, à son extrémité distale, par une paroi d'obturation (19 ou 27).

6. Dispositif selon la revendication 5,
**caractérisé en ce que** ladite paroi d'obturation (19) est pourvue d'un ou de plusieurs orifices (17).

7. Dispositif selon l'une des revendications 5 ou 6,
**caractérisé en ce que** la longueur dudit élément tubulaire (8) est telle que, lorsqu'il est en place dans la trachée dudit patient (1), ladite gaine tubulaire (16) sort de la bouche de celui-ci et **en ce que** la partie de ladite gaine (16), extérieure audit patient, est également pourvue d'orifices (17).

8. Dispositif selon l'une des revendications 3 ou 4,
**caractérisé en ce que** ladite gaine tubulaire (16) est ouverte à son extrémité distale et **en ce que** ledit jeu (18) est relié à une source d'un fluide (23).

9. Dispositif selon l'une quelconque des revendications 3 à 8,
**caractérisé en ce que** ledit élément tubulaire (8) comporte un ressort hélicoïdal (26) entourant ladite gaine tubulaire (16).

10. Dispositif selon la revendication 9,
**caractérisé en ce que** ledit ressort est en une matière à mémoire de forme et prend sa forme hélicoïdale lorsqu'il est soumis aux conditions de température régnant dans la trachée.

11. Dispositif selon la revendication 8,
**caractérisé en ce que** ladite âme tubulaire centrale (14) et ladite gaine tubulaire (16) présentent une forme hélicoïdale.

12. Dispositif selon l'une quelconque des revendications 1 à 11, **caractérisé en ce que** l'extrémité distale dudit élément tubulaire (8) est recourbée.

13. Dispositif selon l'une des revendications 1 ou 2, **caractérisé en ce que** ledit élément tubulaire (8) comporte un corps allongé (28) pourvu d'une pluralité de canaux longitudinaux (29 à 33), dont l'un est apte à être relié à ladite conduite d'alimentation auxiliaire (5B) en gaz respiratoire.

14. Dispositif selon la revendication 13, **caractérisé en ce qu'**un desdits canaux longitudinaux est relié à une source de fluide (23).

15. Dispositif selon l'une des revendications 13 ou 14, **caractérisé en ce qu'**un fil déformable (34) est introduit dans l'un desdits canaux longitudinaux, afin de donner sa forme audit élément tubulaire.

## Patentansprüche

1. Vorrichtung zum Ersetzen eines ersten Beatmungstubus (3), der sich bereits in der Luftröhre (2) eines Patienten (1) befindet, durch einen zweiten Beatmungstubus (11), der sich außerhalb des Patienten befindet, wobei der erste Tubus (3) durch mindestens eine Hauptversorgungsleitung (5A) mit Atemunterstützungsgas versorgt wird, **dadurch gekennzeichnet, dass** sie umfasst:
- eine zusätzliche Leitung (5B) zur Versorgung mit Atemgas mit höherem Druck als derjenige der Hauptversorgungsleitung. (5A) ; und
- ein biegsames und längliches rohrförmiges Element (8) mit mindestens einer distalen Öffnung (9) und mindestens einer proximalen Öffnung (10),
• das dazu geeignet ist, in den ersten und zweiten Beatmungstubus (3 und 11) eingeführt zu werden, indem es diesen ermöglicht, auf dem rohrförmigen Element (8) zu gleiten,
• dessen proximales Ende (10) dazu geeignet ist, mit der zusätzlichen Leitung (5B) verbunden zu werden, und
• dessen distales Ende (9) dazu geeignet ist, das Atemgas der zusätzlichen Leitung (5B) abzugeben.

2. Vorrichtung nach Anspruch 1, **dadurch gekennzeichnet, dass** die Beatmungstuben (3 und 11) dazu geeignet sind, auf der zusätzlichen Versorgungsleitung (5B) zu gleiten.

3. Vorrichtung nach einem der Ansprüche 1 oder 2, **dadurch gekennzeichnet, dass** das rohrförmige Element (8) umfasst:
- einen zentralen rohrförmigen Kern (14) mit der proximalen Öffnung (10), die dazu geeignet ist, mit der zusätzlichen Leitung (5B) verbunden zu werden, und mit einer distalen Öffnung (15) zur Abgabe des Atemgases, und
- eine rohrförmige Hülle (16) die den rohrförmigen Kern (14) mit Spiel (18) umgibt und fest mit dem rohrförmigen Kern (14) verbunden ist.

4. Vorrichtung nach Anspruch 3, **dadurch gekennzeichnet, dass** die Hülle (16) an ihrem distalen Ende in Bezug auf das distale Ende (15) des rohrförmigen Kerns (14) vorsteht.

5. Vorrichtung nach einem der Ansprüche 3 oder 4, **dadurch gekennzeichnet, dass** die rohrförmige Hülle (16) in ihrer Seitenwand mit Öffnungen (17) versehen ist und an ihrem distalen Ende durch eine Verschlusswand (19 oder 27) verschlossen ist.

6. Vorrichtung nach Anspruch 5, **dadurch gekennzeichnet, dass** die Verschlusswand (19) mit einer oder mehreren Öffnungen (17) versehen ist.

7. Vorrichtung nach einem der Ansprüche 5 oder 6, **dadurch gekennzeichnet, dass** die Länge des rohrförmigen Elements (8) derart ist, dass, wenn es sich in der Luftröhre des Patienten (1) befindet, die rohrörmige Hülle (16) aus dessen Mund herausragt, und dass der Teil der Hülle (16), der sich außerhalb des Patienten befindet, ebenfalls mit Öffnungen (17) versehen ist.

8. Vorrichtung nach einem der Ansprüche 3 oder 4, **dadurch gekennzeichnet, dass** die rohrförmige Hülle (16) an ihrem distalen Ende offen ist und dass das Spiel (18) mit einer Fluidquelle (23) verbunden ist.

9. Vorrichtung nach einem der Ansprüche 3 bis 8, **dadurch gekennzeichnet, dass** das rohrförmige Element (8) eine Schraubenfeder (26) umfasst, die die Hülle (16) umgibt.

10. Vorrichtung nach Anspruch 9, **dadurch gekennzeichnet, dass** die Feder aus einem Material mit Formgedächtnis gebildet ist und ihre schraubenförmige Form annimmt, wenn sie den in der Luftröhre herrschenden Temperaturbedingungen ausgesetzt wird.

11. Vorrichtung nach Anspruch 8, **dadurch gekennzeichnet, dass** der zentrale rohrförmige Kern (14) und die rohrförmige Hülle (16) eine schraubenförmige Form aufweisen.

12. Vorrichtung nach einem der Ansprüche 1 bis 11, **dadurch gekennzeichnet, dass** das distale Ende des rohrförmigen Elements (8) gekrümmt ist.

13. Vorrichtung nach einem der Ansprüche 1 oder 2, **dadurch gekennzeichnet, dass** das rohrförmige Element (8) einen länglichen Körper (28) umfasst, der mit einer Vielzahl von in Längsrichtung verlaufenden Kanälen (29 bis 33) versehen ist, von denen einer dazu geeignet ist, mit der zusätzlichen Leitung (5B) zur Versorgung mit Atemgas verbunden zu werden.

14. Vorrichtung nach Anspruch 13, **dadurch gekennzeichnet, dass** einer der in Längsrichtung verlaufenden Kanäle mit einer Fluidquelle (23) verbunden ist.

15. Vorrichtung nach einem der Ansprüche 13 oder 14, **dadurch gekennzeichnet, dass** ein verformbarer Draht (34) in einen der in Längsrichtung verlaufenden Kanäle eingeführt wird, um dem rohrförmigen Element seine Form zu geben.

## Claims

1. Device for replacing a first respiratory probe (3), already in place in the trachea (2) of a patient (1), with a second respiratory probe (11) external to said patient, said first probe (3) being supplied with respiratory assistance gas via at least one main supply conduit (5A), **characterized in that** it includes:
- an auxiliary conduit (5B) for supplying respiratory gas at a higher pressure than that of said main supply conduit (5A); and
- a tubular element (8), flexible and elongate, having at least one distal orifice (9) and at least one proximal orifice (10):
· which is capable of being introduced into said first and second respiratory probes (3 and 11) whilst allowing the latter to slide on said tubular element (8),
· the proximal end (10) of which is capable of being connected to said auxiliary conduit (5B), and
· the distal end (9) of which is capable of dispensing the respiratory gas from said auxiliary conduit (5B).

2. Device according to Claim 1, **characterized in that** said respiratory probes (3 and 11) are capable of sliding on said auxiliary supply conduit (5B).

3. Device according to either of Claims 1 and 2, **characterized in that** said tubular element (8) includes:
- a central tubular core (14) having the proximal orifice (10) which can be connected to said auxiliary supply conduit (5B) and a distal orifice (15) for dispensing said respiratory gas; and
- a tubular sheath (16) surrounding said tubular core (14) with clearance (18) and made integral with said tubular core (14).

4. Device according to Claim 3, **characterized in that**, at its distal end, said sheath (16) projects from the distal end (15) of said tubular core (14).

5. Device according to either of Claims 3 and 4, **characterized in that** said tubular sheath (16) is provided with orifices (17) in its side wall and is closed, at its distal end, by a closure wall (19 or 27).

6. Device according to Claim 5, **characterized in that** said closure wall (19) is provided with one or more orifices (17).

7. Device according to either of Claims 5 and 6, **characterized in that** the length of said tubular element (8) is such that when it is in place in the trachea of said patient (1), said tubular sheath (16) protrudes from the patient's mouth, and **in that** that part of said sheath (16) external to said patient is also provided with orifices (17).

8. Device according to either of Claims 3 and 4, **characterized in that** said tubular sheath (16) is open at its distal end, and **in that** said clearance (18) is connected to a source of a fluid (23).

9. Device according to any one of Claims 3 to 8, **characterized in that** said tubular element (8) includes a helical spring (26) surrounding said tubular sheath (16).

10. Device according to Claim 9, **characterized in that** said spring is made of a material with shape memory and assumes its helical shape when it is subjected to the temperature conditions prevailing in the trachea.

11. Device according to Claim 8, **characterized in that** said central tubular core (14) and said tubular sheath (16) have a helical shape.

12. Device according to any one of Claims 1 to 11, **characterized in that** the distal end of said tubular element (8) is curved back.

13. Device according to either of Claims 1 and 2, **characterized in that** said tubular element (8) includes an elongate body (28) provided with a plurality of longitudinal channels (29 to 33), one of which can be connected to said auxiliary supply conduit (5B) for respiratory gas.

14. Device according to Claim 13, **characterized in that** one of said longitudinal channels is connected to a source of fluid (23).

15. Device according to either of Claims 13 and 14, **characterized in that** a deformable wire (34) is introduced into one of said longitudinal channels in order to impart its shape to said tubular element.
